# EUROPEAN PATENT SPECIFICATION

(11) **EP 3 893 992 B1**
(45) Date of publication and mention of the grant of the patent: **10.09.2025**
(21) Application number: 19813888.5
(22) Date of filing: 10.12.2019
(51) Int. Cl.: A61N 5/06, A61K 41/00, A61L 2/00, A61L 2/08, C02F 1/50

(54) **COMPOSITION FOR THE USE IN TOPICAL ANTIMICROBIAL OR ANTI-INFECTIVE TREATMENT OF SKIN, SOFT TISSUE AND WOUNDS**
ZUSAMMENSETZUNG ZUR VERWENDUNG IN DER TOPISCHEN ANTIMIKROBIELLEN ODER ANTIINFEKTIÖSEN BEHANDLUNG VON HAUT, WEICHGEWEBE UND WUNDEN
COMPOSITION DESTINÉE À ÊTRE UTILISÉE DANS LE TRAITEMENT TOPIQUE ANTIMICROBIEN OU ANTI-INFECTIEUX DE LA PEAU, DES TISSUS MOUS ET DES PLAIES

(30) Priority: 11.12.2018 EP 18211776
(43) Date of publication of application: 20.10.2021
(73) Proprietor: bredent medical GmbH & Co. KG, 89250 Senden (DE)
(72) Inventor: VANDER BEKEN, Seppe, 89134 Blaustein (DE); MICKO, Gerald, 88471 Laupheim (DE)
(74) Representative: Baur & Weber Patentanwälte PartG mbB
(86) International application number: PCT/EP2019/084486
(87) International publication number: WO 2020/120500

(56) References cited:
- WO-A1-2016/039812
- COOPER MICHOL A ET AL: "Medical and surgical treatment of chronic venous ulcers", SEMINARS IN VASCULAR SURGERY, ELSEVIER, AMSTERDAM, NL, vol. 28, no. 3, 22 December 2015 (2015-12-22), pages 160 - 164, XP029519664, ISSN: 0895-7967, DOI: 10.1053/J.SEMVASCSURG.2015.12.003

## Description

The invention relates to a composition for the use in topical antimicrobial or anti-infective treatment of skin, soft tissue and wounds.

### Background of the Invention

Devices for photodynamic therapy and respective substances for usage with such devices are known.

For example, in US 2012/0296260 A1 an apparatus for photodynamic therapy and/or for destroying or reducing microorganisms is shown comprising an irradiation unit having at least one light source, by means of which a photosensitizer applied to a wound area to be treated is activated by way of irradiation, further comprising a camera for recording images of the wound, which is disposed in the irradiation unit, and a positioning unit, by means of which the irradiation unit can be oriented with respect to the wound area. On a display for the images is a grid with visual indicia distinguishing fields to be irradiated by means of the light sources.

EP 1 737 492 A1 shows a preparation for the photodynamic control of micro-organisms, said preparation being in a liquid or pasty form and containing a photosensitizer which comprises a dyestuff and produces singlet oxygen when irradiated by means of light. The micro-organisms can be marked by means of the dyestuff. The aim is to improve said preparation in order to enable an improved photodynamic control. To this end, the preparation contains an active ingredient for amplifying or weakening the oxidative action of the singulet oxygen by chemical manipulation of the dyestuff or the nano environment thereof. In a particular form, a rinsing solution is used before the irradiation.

More generally other forms of substances for wound treatment are known.

In DE 100 12 026 A1 a gel is described which comprises an aqueous solution containing (by weight) 0.01-0.3% polyhexamethylene biguanide (PHMB), 1-15 wt. % glycerol and 0.2-5% hydroxyethyl cellulose (HEC).

In DE 10 2004 037 598 A1 a medium for use in mouth and throat area is described, which comprises microbicide aqueous solution comprising a linear biguanide polymer and/or water-soluble salt of microbicide in combination with at least a sweetener.

WO 2003/004013 A1 shows a wound treatment agent that contains, in aqueous solution, polyhexamethylene biguanide and at least one surfactant. The surfactant is a glycine derivative and/or a sulfosuccinate and/or an amide based on an unbranched fatty acid. The surfactant is preferably a betaine and, in particular, an amidoalkyl betaine of a fatty acid. The wound treatment agent is excellently suited for use as a washing or shower gel, as a moisturizing gel or as a moist wound covering, as a dissolving gel for dissolving incrustations or scabs from body surfaces or wounds or for removing dressings and for changing moist dressings.

In EP 1 263 465 B1 a method of photoeradication of cellular and acellular organisms is shown which includes the steps of providing a surface acting agent in association with a cellular or acellular organism, the surface acting agent disorienting a membrane structure so that said membrane no longer functions as an effective osmotic barrier; providing a photosensitive material in association with the cellular or acellular organism; and applying light in association with the cellular or acellular organism to cause a disruption of the organism.

WO 2010/070292 A1 shows a composition for use on skin and wounds, comprising a photo-catalyst which is capable of preferentially staining biofilms the composition being for use in the diagnosis and treatment of biofilms in wounds.

In WO 2005/034855 A1, the use of photosensitizers is disclosed so as to inactivate bacterial spores of bacterial species including Bacillus anthracis, which is useful in the decontamination and treatment of living animals and in the decontamination of inanimate objects and substances.

WO 2016/039812 A1 shows compositions for the photodynamic control of micro- organisms wherein the compositions comprise a photosensitiser which comprises a dyestuff and produces singlet oxygen when irradiated by means of light for the disinfection and sterilization of materials, commodities and surfaces contaminated with one or more species of micro-organisms including bacteria, fungi, algae, yeasts, bacterial spores and fungal spores.

### Summary of the Invention

It is therefore an object of the invention to provide a composition which safely enhances the antimicrobial effect of antiseptics and antimicrobial photodynamic therapy for disinfection therapy.

This object is addressed by independent claim 1. Further advantageous embodiments of the invention are described in the sub-claims. These can be combined in technologically meaningful ways. The specification additionally characterizes and specifies the invention.

According to the invention, a composition for the use in topical antimicrobial or anti-infective treatment of skin, soft tissue and wounds is described, said composition comprising polyhexamethylene biguanide (PHMB) as an antiseptic substance and methylene blue (MB) as a photosensitizer and said composition is provided as a biocompatible and healing-supportive hydrogel at the time of application, wherein the antiseptic substance exerts an antimicrobial or anti-infective effect on microorganisms or their biofilm on skin, soft tissue and wounds and the photosensitizer catalyzes a photodynamic antimicrobial or anti-infective effect upon irradiation with light of appropriate wavelength, power-density and energy-dose, directed at the microorganisms or their biofilm on skin, soft tissue and wounds, wherein the combined antimicrobial or anti-infective effects work additive or synergistically to achieve a therapeutically significant grade of disinfection.

Accordingly, the simultaneous application of both the antiseptic substance and the photosensitizer in a hydrogel and the combined corresponding skin, soft tissue and wounds antimicrobial therapy-modalities is therapeutically advantageous over application of the antiseptic substance and the photosensitizer with subsequent photodynamic therapy alone.

The advantage is achieved for one or more of the following reasons by the combined therapy. The combined application of the antiseptic substance and the photosensitizer with subsequent antimicrobial photodynamic therapy results in a broader antimicrobial and or anti-infective spectrum. An additive or synergistic antimicrobial or anti-infective effect on microorganisms and their biofilm possibly causing the skin and soft tissue infections can be achieved by the combined application of the antiseptic substance and the photosensitizer-mediated antimicrobial photodynamic therapy, which also reduces the risk for tolerance- or resistance-development by the targeted microorganisms. A reduction of the effective concentration of both substances can be achieved in the inventive composition, which lowers the risk of unwanted side effects by the respective substances on the patient.

Although the effect and some interactions of the essential ingredients of the inventive composition are known, they are advantageously combined in the novel triple combination (i.e. hydrogel-PHMB-MB) for use in modern moist and phase-specific wound treatment. The composition can be applied to any type of skin wound and offers different therapy or care options depending on the wound condition (clinical picture): moisture regulation and MB-assisted autolytic debridement to promote wound healing, assistance for the ink-supported mechanical wound cleaning, and PHMB-supported wound disinfection using MB-aPDT. Effect of the inventive composition is hydrogel for modern moist wound treatment for moisture regulation, autolytic wound debridement and to promote wound healing. Hydrogels contain a lot of water, which is released in a dry wound environment, and polymeric substances, which form a breathable protective film on the wound. MB for color-supported wound cleansing (visual aid) and as a photo-sensitive substance for wound disinfection with aPDT. PHMB as well-tolerated preservative with antimicrobial effect, regulates the microbiological balance in the wound.

Interactions between hydrogel and PHMB (up to 0.1%) is that both stimulate wound healing, PHMB prevents microbial contamination of the gel (preservative), prevents microbial growth in the wound and infections: creation of a microbial balance of the wound environment. Between MB-aPDT and PHMB an additive or synergetic effect in wound disinfection can be achieved. Between hydrogel and MB; MB supports autolytic debridement of wounds by hydrogel (= resolving and removal of wound debris) by molecular binding to fibrin, non-vital (necrotic) tissue and biofilm and improved absorption by secondary wound dressings, whereas hydrogel has a cooling effect on the skin that counteracts possible warming of the tissue during MB-aPDT because of light absorption.

Using PHMB and MB for the two substances of the inventive composition is preferred and achieves the therapeutically advantageous results. More generally, the class of phenothiazinium photosensitizers is well studied, documented and approved of in topical applications for antimicrobial photodynamic therapy and comprises the dyes MB and its derivatives, including new methylene blue and 1,9-dimethyl-methylene blue, as well toluidine blue O (TBO) and its derivatives.

Antiseptic substances are generally used and well established in clinical practice. Presently these substances are standard of care as a stand-alone product for the prevention and treatment of critical colonization and infection of skin, soft tissue and wounds with pathogenic microorganisms. These substances include the mentioned PHMB, while other known antiseptic substances, indicated for antimicrobial or anti-infective treatment of skin, soft tissue and wounds, could be used as well. PHMB has, among wound antiseptic substances, a preferred safety profile with good biocompatibility and wound healing supportive effects.

In this respect, the antiseptic substance (PHMB) of the composition is provided with a concentration below a biocompatible maximum concentration threshold. The concentration of the antiseptic substance (PHMB) can be up to 0.1 %.

According to further embodiments of the invention, both the antiseptic substance and the photosensitizer each are present in an amount, sufficient to have or mediate an antimicrobial or anti-infective effect. Both the antiseptic substance and the photosensitizer each are advantageously present in an amount below their respective toxicity levels for host animal/human cells and tissue, which provides for therapeutically advantageous results without side effects or damages.

According to the invention, the composition is provided as a hydrogel or an aquagel. In this respect, the composition can be further comprising a polymeric gelling substance and water. This is in particular advantageous, when the composition is directly applied during treatment, for example in autolytic wound debridement and/or photodynamic therapy. In other embodiments, the composition according to the invention can also be embedded into a medical dressing or a patch.

The composition can further comprise substances for modifying the physical and physiochemical properties of the formulation, including pH, tonicity, stability, preservation, viscosity, surface-activity and emulsification as well as skin permeation enhancers, slow-release- or small molecule-carriers, humectants and antioxidants and/or substances for neutralizing bad odors caused by skin, soft tissue and wound infections. Adding such further substances enhances the application of the composition.

The use of the inventive composition in topical antimicrobial or anti-infective treatment can be extended to the application to body orifices and associated mucosae. Advantageously, the oral cavity including tongue, upper throat, tonsils, gum, gingiva, teeth and implants, as well as the nose, the auditory canal can be treated in addition or instead of bodily external skin areas.

In this case the composition may further comprise a substance for modifying taste or sweetness of the composition.

Furthermore, the composition may be used with respect to anorectal and genital mucosae.

It is particularly advantageous using the above-described composition in photodynamic therapy utilizing at least one wavelength being selected in the range of the absorption maximum of the photosensitizer and utilizing power density and energy dose ranges to allow for an antimicrobial or anti-infective effect mediated by the photosensitizer without causing damage to the skin, soft tissue or wound.

According to another embodiment of the invention, the use of the inventive composition can be extended to non-topical antimicrobial or anti-infective treatment applications including by ingestion, by injections or by instillation in surgically created openings to the body.

According to yet another embodiment of the invention, the use of the inventive composition can be extended to industrial antimicrobial or anti-infective applications including food preservation, decontamination of foodstuff, decontamination of aquaculture-, waste- and drinking-water as well as disinfection of surfaces and devices.

### Detailed Description of the Invention

In the following, embodiments of the invention will be explained in more detail.

The invention refers to a composition for the use in topical antimicrobial or anti-infective treatment of skin, soft tissue and wounds.

The composition comprises an antiseptic substance and a photosensitizer, provided simultaneously as a hydrogel at the time of application.

In general, skin, soft tissue and wound infections refer to the critical colonization or infection with potentially pathogenic microorganisms and their biofilm of the skin, skin wound or ulcer and underlying soft tissues, leading to infectious disease and symptoms including tissue inflammation and pain, stalled wound healing or even tissue damage and necrosis.

An antimicrobial effect, i.e. the killing of potentially pathogenic microorganisms or destruction of its biofilm, and anti-infective effect or the prevention of serious local spreading and systemic infection with such microorganisms, is advantageous for the patient for reasons that include enhanced wound healing or infective disease prevention and resolution.

A broad antimicrobial spectrum and efficiency and at the same time a low toxicity to the treated skin, soft tissue or wound are advantageous characteristics of a composition for antimicrobial or anti-infective treatment.

The antiseptic substance exerts an antimicrobial or anti-infective effect on microorganisms or their biofilm causing skin and soft tissue infections. The antiseptic substance can be polyhexamethylene biguanide (PHMB), but alcohols (ethanol, isopropanol, etc.), quaternary ammonium compounds (benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride and domiphen bromide), chlorhexidine gluconate, octenidine dihydrochloride, hydrogen peroxide, sodium hypochlorite, hypochlorous acid, chloroxylenol, ethacridine lactate, iodophores or antiseptic solutions like electrochemically activated water (ECA) could be used as well. Advantageously, the antiseptic substance absorbs as less light as possible at the wavelengths used for simultaneous photodynamic therapy with the inventive composition or does not chemically abrogate the photo-catalyzing properties of the photosensitizer when combined within the inventive composition and can technically be incorporated in a hydrogel.

The photosensitizer catalyzes a photodynamic antimicrobial or anti-infective effect upon irradiation with light of appropriate wavelength, power-density and energy-dose, directed at the microorganisms or their biofilm possibly causing skin, soft tissue and wound infections.

Typically, phenothiazinium-class photosensitizers are the well-studied antimicrobial dye-type photosensitizers for topical use that could be advantageously used in combination with an antiseptic substance. However, other such synthetic dye antimicrobial photosensitizers (e.g. rose bengal, erythrosine, indocyanine green, crystal violet) as well as other classes of antimicrobial photosensitizers, such as cyclic polypyrroles (e.g. porphyrins, chlorins, bacteriochlorins, phthalocyanines) and other natural (e.g. psoralens, perylenequinones, curcumin, riboflavin) or synthetic (e.g. boron-dipyrromethene and squaraine-based chromophores, fullerenes) photoactive compounds could be used as well. Phenothiazinium-class photosensitizers comprise the preferred dye methylene blue (MB) and its derivatives, including new methylene blue and 1,9-dimethyl-methylene blue, as well toluidine blue O and its derivatives.

The combined application of both the antiseptic substance and the photosensitizer for simultaneously performing both corresponding antimicrobial or anti-infective therapy-modalities for skin, soft tissue and wound infections are therapeutically advantageous over each single modality alone.

First, a broader antimicrobial and or anti-infective spectrum can be achieved. Each single antimicrobial or anti-infective therapeutic modality is associated with a specific antimicrobial spectrum that may largely overlap but also shows differences in efficacy against certain types of microorganisms. The simultaneous application of both modalities therefore has an extended antimicrobial spectrum as compared to each one of the single modalities applied alone.

Second, an additive or synergistic antimicrobial or anti-infective effect on microorganisms and their biofilm on the skin, soft tissue and wounds can be achieved. Both substances can sensitize microorganisms for the other antimicrobial or anti-infective modality, respectively, by improving bioavailability and/or localization of the other substance to said microorganisms and their biofilm. This causes an improved effectiveness by the combined-modalities application and reduces the risk for tolerance- or resistance-development by microorganisms causing the skin and soft tissue infections to each said single therapy-substance. As an example, this is especially important for gram-negative pathogenic bacteria, such as Pseudomonas aeruginosa, a bacterial species often associated with biofilms found in hard to heal wounds. Pseudomonas aeruginosa infections are difficult to treat with either PHMB or MB-mediated photodynamic therapy alone but are less tolerant to the combined antimicrobial treatment approach.

Third, a reduction of the effective concentration of both substances needed in the composition can be achieved, which lowers the risk of unwanted side effects by the respective substances on the patient. Overall, the benefit-over-risk ratio improves, meaning that the therapeutic efficiency increases whilst unwanted side effects can be reduced or prevented. As an example, the preferred antiseptic substance PHMB was declared safe, also for cosmetic applications, up to a concentration of 0.1% by the European Commission - Scientific Committee on Consumer Safety. To achieve a therapeutically sufficient antimicrobial effect in a shorter time on readily infected wounds, especially with (vancomycin-resistant) Enterococcus or Pseudomonas aeruginosa, wound disinfection products with PHMB generally require a higher amount than 0.1% of this antiseptic substance, which thus exceeds the consensus biocompatible maximum concentration. This can be overcome with the current invention by combining low-dose PHMB (up to 0.1%) with a Photosensitizer for simultaneous aPDT.

Typically, the composition is provided as a hydrogel or an aquagel and further comprises a polymeric gelling substance and water. Accordingly, the composition can be directly applied during treatment, for example in autolytic wound debridement and/or photodynamic therapy. It is however also possible embedding the composition into a medical dressing or a patch.

Typically, polymeric gelling substances for topical applications are natural proteins (e.g. gelatin, casein, collagen, egg whites, polysaccharides), semisynthetic cellulose subordinates (e.g. carboxymethyl cellulose, ethylcellulose, hydroxyethylcellulose, hydroxypropyl cellulose, hydroxypropylmethyl cellulose, Mg/Al-silicate, methylcellulose, sodium alginate) or fully synthetic carbomers, poloxamers, polyvinyl alcohols and others.

The composition can further comprise substances for modifying the physical and physiochemical properties of the formulation, including pH, tonicity, stability, preservation, viscosity, surface-activity and emulsification as well as skin permeation enhancers, slow-release- or small molecule-carriers, humectants and antioxidants and/or substances for neutralizing bad odors caused by skin and soft tissue infections. Adding such further substances enhances the application of the composition.

The use of the composition in topical antimicrobial or anti-infective treatment can be extended to the application to body orifices and associated mucosae. Advantageously, the oral cavity including tongue, upper throat, tonsils, gum, gingiva, teeth and implants, as well as the nose, the auditory canal can be treated in addition or instead of skin areas. In this case the composition may further comprise a substance for modifying taste or sweetness of the composition. Furthermore, the composition may be used with respect to anorectal and genital mucosae.

According to another embodiment of the invention, the use of the inventive composition can be extended to non-topical antimicrobial or anti-infective treatment applications including by ingestion, by injections or by instillation in surgically created openings to the body. In particular, instillation into a surgically created opening can be advantageous for antimicrobial treatment or disinfection of cysts or implant infections. Hereto, the composition as well as the optical device or probe for light application for applying the photodynamic therapy and that enter the body need to be sterile.

According to yet another embodiment of the invention, the use of the inventive composition can be extended to industrial antimicrobial or anti-infective applications including food preservation, decontamination of foodstuff, decontamination of aquaculture-, waste- and drinking-water as well as disinfection of surfaces and devices. These represent novel domains in which the application of antimicrobial photodynamic therapy is potentially advantageous for disinfection purposes.

The combined application of a hydrogel composition comprising both a photosensitizer for photodynamic treatment and an antiseptic substance at the time of application for said extended medical and industrial uses can be advantageous for the same reasons as for the antimicrobial or anti-infective topical application to skin, soft tissue or wounds.

In the following, experimental results are provided. In vitro experiments to kill Pseudomonas aeruginosa germs in a wound-representative semi-solid biofilm model have been performed. In this respect setup A refers to no treatment at all. Setup B and C use either hydrogel with low-dose PHMB as the antiseptic substance or hydrogel with low-dose MB as the photosensitizer. Setup D refers to the invention, where both low-dose PHMB as the antiseptic substance and low-dose MB as the photosensitizer are used simultaneously and combined in hydrogel.

For setup C and D, i.e. those utilizing a photosensitizer, the respective light irradiations have been performed with the same values for wavelength, power-density and energy-dose. For all experimental setups, the fractions of gelling substance and water have been adapted so as to arrive at the same viscosity. The survival rate of the microorganisms is expressed in the usual terms of colony-forming units (CFU).

The results, illustrating the significant additive or synergistic antimicrobial effect of the combined test setup D as intended by the invention as compared to the single test setups B (low-dose PHMB) or C (low-dose MB-aPDT) are summarized in the following table:

**Table: Statistical analysis that revealed significant differences in bacterial counts between experimental setups performed in triplicate (Welch's t-test p-value < 0.05) are annotated with a different superscript small letter a, b or c.**

| Setup | % PHMB | % MB | Mean CFU/well (± SD), relative to A |
|---|---|---|---|
| A | 0 | 0 | 100% (± 24.1%)^{a} |
| B | 0.1 | 0 | 19.7% (± 9.7%)^{b} |
| C | 0 | 0.02 | 28.3% (± 6.2%)^{b} |
| D | 0.1 | 0.02 | 1.8% (± 1.6%)^{c} |

The inventive composition can be used together with a therapy device for irradiating a surface of a tissue, in particular skin tissue.

The invention is defined in the following claims. Other embodiments, examples, methods etc. are not a part of the application and are provided for illustrative purposes only.

## Claims

1. A composition for the use in topical antimicrobial or anti-infective treatment of skin, soft tissue and wounds, said composition comprising polyhexamethylene biguanide, PHMB, as an antiseptic substance and methylene blue, MB, as a photosensitizer and wherein said composition is provided as a biocompatible and healing-supportive hydrogel at the time of application, wherein the PHMB exerts an antimicrobial or anti-infective effect on microorganisms or their biofilm on skin, soft tissue and wounds and MB catalyzes a photodynamic antimicrobial or anti-infective effect upon irradiation with light of appropriate wavelength, power-density and energy-dose, directed at the microorganisms or their biofilm on skin, soft tissue and wounds,
wherein both the PHMB and the MB each are present in an amount sufficient to have an antimicrobial or anti-infective effect and said antimicrobial or anti-infective effects combined work additive or synergistically to achieve a therapeutically significant grade of disinfection, wherein the concentration of PHMB is up to 0.1%.

2. The composition according to claim 1, wherein PHMB is provided with a concentration below a biocompatible maximum concentration threshold.

3. The composition according to claim 1 or 2, wherein the concentration of MB is up to 0.02%.

4. The composition according to any of claims 1 to 3, wherein both PHMB and MB each are present in concentration below their respective maximum biocompatible concentration threshold for host animal/human cells and tissue.

5. The composition according to any of claims 1 to 4, wherein the hydrogel is comprising a polymeric gelling substance and water.

6. The composition according to any of claims 1 to 5, which is provided within a medical dressing device or a patch.

7. The composition according to any of claims 1 to 6, which further comprises substances for modifying the physical and physiochemical properties of the formulation, including pH, tonicity, stability, preservation, viscosity, surface-activity and emulsification as well as skin permeation enhancers, slow-release- or small molecule carriers humectants and antioxidants, and/or substances for neutralizing bad odors caused by skin and soft tissue infections.

8. The composition according to any of claims 1 to 7, which is used in topical antimicrobial or anti-infective treatment being extended to the application to body orifices and associated mucosae.

9. The composition according to claim 8, further comprising a substance for modifying taste or sweetness of the composition.

## Patentansprüche

1. Zusammensetzung zur Verwendung bei der örtlichen antimikrobiellen oder antiinfektiösen Behandlung von Haut, Weichgewebe und Wunden, wobei die Zusammensetzung Polyhexamethylenbiguanid, PHMB, als antiseptische Substanz und Methylenblau, MB, als Photosensibilisator umfasst und wobei die Zusammensetzung zum Zeitpunkt der Anwendung als biokompatibles und heilungsunterstützendes Hydrogel bereitgestellt wird, wobei das PHMB eine antimikrobielle oder antiinfektiöse Wirkung auf Mikroorganismen oder deren Biofilm auf der Haut ausübt, eine antimikrobielle oder antiinfektiöse Wirkung auf Mikroorganismen oder deren Biofilm auf Haut, Weichgewebe und Wunden ausübt und MB eine photodynamische antimikrobielle oder antiinfektiöse Wirkung bei Bestrahlung mit Licht geeigneter Wellenlänge, Leistungsdichte und Energiedosis katalysiert, die auf die Mikroorganismen oder deren Biofilm auf Haut, Weichgewebe und Wunden gerichtet ist, wobei sowohl das PHMB als auch das MB jeweils in einer Menge vorhanden sind, die ausreicht, um eine antimikrobielle oder antiinfektiöse Wirkung zu haben, und die antimikrobiellen oder antiinfektiösen Wirkungen kombiniert additiv oder synergistisch wirken, um einen therapeutisch bedeutsamen Desinfektionsgrad zu erreichen, wobei die Konzentration des PHMB bis zu 0.1% ist.

2. Zusammensetzung nach Anspruch 1, wobei PHMB in einer Konzentration unterhalb einer biokompatiblen Höchstkonzentrationsschwelle bereitgestellt wird.

3. Zusammensetzung nach Anspruch 1 oder 2, wobei die Konzentration von MB bis zu 0,02 % beträgt.

4. Zusammensetzung nach einem der Ansprüche 1 bis 3, wobei sowohl PHMB als auch MB jeweils in einer Konzentration unterhalb ihrer jeweiligen maximalen biokompatiblen Konzentrationsschwelle für tierische/menschliche Wirtszellen und Gewebe vorliegen.

5. Zusammensetzung nach einem der Ansprüche 1 bis 4, wobei das Hydrogel eine polymere Geliersubstanz und Wasser enthält.

6. Zusammensetzung nach einem der Ansprüche 1 bis 5, die in einer medizinischen Verbandvorrichtung oder einem Pflaster bereitgestellt wird.

7. Zusammensetzung nach einem der Ansprüche 1 bis 6, die ferner Substanzen zur Veränderung der physikalischen und physiochemischen Eigenschaften der Formulierung, einschließlich pH-Wert, Tonizität, Stabilität, Konservierung, Viskosität, Oberflächenaktivität und Emulgierung, sowie Hautpermeationsverstärker, Trägermittel mit langsamer Freisetzung oder kleine Moleküle, Feuchthaltemittel und Antioxidantien und/oder Substanzen zur Neutralisierung schlechter Gerüche, die durch Haut- und Weichteilinfektionen verursacht werden, enthält.

8. Zusammensetzung nach einem der Ansprüche 1 bis 7, die zur örtlichen antimikrobiellen oder antiinfektiösen Behandlung verwendet wird, wobei die Anwendung auf Körperöffnungen und zugehörige Schleimhäute ausgedehnt wird.

9. Zusammensetzung nach Anspruch 8, die ferner eine Substanz zur Modifizierung des Geschmacks oder der Süße der Zusammensetzung enthält.

## Revendications

1. Composition à utiliser dans un traitement topique antimicrobien ou anti-infectieux de la peau, des tissus mous et des plaies, ladite composition comprenant du polyhexaméthylène biguanide, PHMB, en tant que substance antiseptique et du bleu de méthylène, MB, en tant que photosensibilisateur et dans laquelle ladite composition est fournie sous forme d'hydrogel biocompatible et favorisant la cicatrisation au moment de l'application, dans laquelle le PHMB exerce un effet antimicrobien ou anti-infectieux sur les micro-organismes ou leur biofilm sur la peau, les tissus mous et les plaies et le MB catalyse un effet antimicrobien ou anti-infectieux photodynamique lors de l'irradiation avec une lumière de longueur d'onde, de densité de puissance et de dose d'énergie appropriées, dirigée vers les micro-organismes ou leur biofilm sur la peau, les tissus mous et les plaies, dans laquelle le PHMB et le MB sont tous deux présents en une quantité suffisante pour avoir un effet antimicrobien ou anti-infectieux et lesdits effets antimicrobiens ou anti-infectieux combinés agissent de manière additive ou synergique pour atteindre un niveau de désinfection thérapeutiquement significatif, dans laquelle la concentration de PHMB est inférieure ou égale à 0,1 %.

2. Composition selon la revendication 1, dans laquelle le PHMB est fourni à une concentration inférieure à un seuil de concentration maximale biocompatible.

3. Composition selon la revendication 1 ou 2, dans laquelle la concentration de MB est inférieure ou égale à 0,02 %.

4. Composition selon l'une quelconque des revendications 1 à 3, dans laquelle le PHMB et le MB sont tous deux présents en concentration inférieure à leur seuil de concentration maximale biocompatible respectif pour les cellules et tissus animaux/humains hôtes.

5. Composition selon l'une quelconque des revendications 1 à 4, dans laquelle l'hydrogel comprend une substance gélifiante polymère et de l'eau.

6. Composition selon l'une quelconque des revendications 1 à 5, qui est fournie dans un dispositif de pansement médical ou un timbre.

7. Composition selon l'une quelconque des revendications 1 à 6, qui comprend en outre des substances destinées à modifier les propriétés physiques et physico-chimiques de la formulation, y compris le pH, la tonicité, la stabilité, la conservation, la viscosité, l'activité de surface et l'émulsification, ainsi que des activateurs de perméation cutanée, des transporteurs à libération lente ou de petites molécules, des humectants et des antioxydants, et/ou des substances destinées à neutraliser les mauvaises odeurs causées par les infections de la peau et des tissus mous.

8. Composition selon l'une quelconque des revendications 1 à 7, qui est utilisée dans un traitement topique antimicrobien ou anti-infectieux s'étendant à l'application sur les orifices corporels et les muqueuses associées.

9. Composition selon la revendication 8, comprenant en outre une substance destinée à modifier le goût ou la douceur de la composition.
